# EUROPÄISCHE PATENTSCHRIFT

(11) **EP 2 689 204 B1**
(45) Veröffentlichungstag und Bekanntmachung des Hinweises auf die Patenterteilung: **27.02.2019**
(21) Anmeldenummer: 12711827.1
(22) Anmeldetag: 23.03.2012
(51) Int. Cl.: F27B 9/24, F27B 9/30, F27D 7/02, F27D 99/00

(54) **ROLLENHERDOFEN UND VERFAHREN ZUM ERWÄRMEN VON WERKSTÜCKEN**
ROLLER HEARTH FURNACE AND METHOD FOR HEATING WORKPIECES
FOUR À ROULEAUX ET PROCÉDÉ DE CHAUFFAGE DE PIÈCES

(30) Priorität: 25.03.2011 DE 102011006171
(43) Veröffentlichungstag der Anmeldung: 29.01.2014
(73) Patentinhaber: Schwartz GmbH, 52152 Simmerath (DE)
(72) Erfinder: SCHWARTZ, Rolf-Josef, 52152 Simmerath (DE)
(74) Vertreter: KNH Patentanwälte Neumann Heine Taruttis PartG mbB
(86) Internationale Anmeldenummer: PCT/EP2012/055188
(87) Internationale Veröffentlichungsnummer: WO 2012/130750

(56) Entgegenhaltungen:
- DE-U1-202005 006 587
- JP-A- 6 185 876
- JP-A- 7 090 352
- JP-A- 2000 109 926
- JP-A- 2009 030 848
- JP-A- 2010 043 816
- US-B1- 7 520 746

## Beschreibung

Die Erfindung betrifft einen Rollenherdofen zum Erwärmen von Werkstücken. Dieser Rollenherdofen umfasst mindestens eine Ofenkammer zur Aufnahme der Werkstücke mit mindestens zwei seitlichen Ofenwänden, wobei innerhalb der Ofenkammer wenigstens eine Transportrolle zum Transport der Werkstücke durch die Ofenkammer angeordnet ist. Die Ofenwände weisen jeweils wenigstens eine Öffnung auf, durch welche die Transportrolle hindurchführbar ist und an einer Außenseite der Ofenwände ist jeweils mindestens eine Wälzlagereinheit angeordnet, die dazu ausgebildet ist, die Transportrolle außerhalb der Ofenkammer aufzunehmen und zu lagern.

Die Erfindung betrifft ferner ein Verfahren zum Erwärmen von Werkstücken, bei dem ein solcher Rollenherdofen eingesetzt wird.

In der Industrie kommen in zunehmendem Maße Stahlbauteile zur Anwendung, die ein möglichst günstiges Verhältnis von Festigkeit zu Gewicht aufweisen sollen. Das kann beispielsweise durch den Prozess des so genannten Presshärtens oder auch Formhärtens erreicht werden. Dabei wird ein Blechteil auf etwa 800°C bis 1000°C erwärmt und anschließend in einem gekühlten Werkzeug verformt und abgeschreckt. Die Festigkeit des Blechteils nimmt dadurch bis auf etwa ein Dreifaches zu. Presshärten ermöglicht den Bau leichter und dennoch steifer Stahlbauteile durch die Kombination von Wärmebehandlung und Formgebung bei gleichzeitiger kontrollierter Abkühlung. Neben dem Presshärten von Stahlbauteilen gibt es in der Industrie aber auch noch eine Vielzahl anderer Prozesse, bei denen ein Erwärmen von Werkstücken in einem Ofen erforderlich ist, wie beispielsweise Vergüten oder Härten von Werkstücken.

Für die Wärmebehandlung der Stahlbauteile haben sich in der Vergangenheit verschiedene Ofenkonzepte herausgebildet. Insbesondere das Prinzip des Durchlaufofens hat beim Presshärten große Verbreitung gefunden. Bei diesem Ofenprinzip werden die zu erwärmenden Stahlbauteile über Fördereinrichtungen durch den Ofen transportiert. Derartige Durchlauföfen weisen üblicherweise eine oder auch mehrere Ofenkammern zum Erwärmen der Stahlbauteile auf, in denen sich ein Ofengas befindet und in welche direkt oder indirekt die von Heizelementen abgegebene Wärme eingeleitet wird. Den Transport der zu erwärmenden Stahlbauteile durch den Ofen übernimmt eine Fördereinrichtung. Als Fördereinrichtung kommen dabei sehr häufig Rollen in Form eines Rollenförderers zum Einsatz.

Die Rollen des Rollenförderers werden jeweils auf beiden Seiten des Durchlaufofens durch Öffnungen in der Ofenwand der Ofenkammer hindurch geführt und außerhalb der Ofenkammer in Wälzlagern gelagert, die üblicherweise als Kugellager ausgebildet und an der Außenseite der Ofenwand angeordnet sind. Da die Temperatur an der Außenseite der Ofenwand der Ofenkammer nur etwa 100°C beträgt, können Kugellager mit Schmierstoffen im Inneren zum Einsatz kommen, die die Laufeigenschaften der Kugellager verbessern sowie deren Lebensdauer mitbestimmen.

Während des Betriebs des Rollenherdofens führen Verschmutzungen des Schmierstoffes und/oder Verringerungen der Schmierstoffmenge allerdings häufig zu einer Reduzierung der Lebensdauer der Kugellager für die Rollen. Ursache dafür kann beispielsweise aus dem Rollenherdofen austretendes Ofengas sein, das durch die Öffnungen in der Ofenwand aus der Ofenkammer nach außen gelangt und dabei auch durch die an der Außenseite der Ofenwand angeordneten Kugellager strömt. Dabei werden die während der Rollendrehungen und der damit verbundenen Reibung an der keramischen Isolierwand im Ofen entstehenden Partikel durch das Ofengas teilweise mit nach außen befördert und beim Durchströmen in die Kugellager eingetragen. Bedingt durch die hohe Temperatur des Ofengases wird außerdem der Schmierstoff langsam aufgelöst, sodass sich die Schmierstoffmenge zeitabhängig entsprechend reduziert.

Sowohl die Verunreinigungen des Schmierstoffes durch den Partikeleintrag als auch die Mengenreduzierung durch das heiße Ofengas erhöhen den Verschleiß der Kugellager für die Rollen und wirken sich somit nachteilig im Hinblick auf die Lebensdauer der Kugellager aus.

Die japanische Offenlegungsschrift JP-A 2000 109926 offenbart eine Vorrichtung sowie ein Verfahren zur Abdichtung der Rollendurchführungen eines Rollenherdofens. Die Vorrichtung weist äußere und innere Stopfbuchseneinheiten auf. Die Rollen erstrecken sich durch Löcher in den Seitenwänden des Ofens in der Ofenkammer nach außen. Jedes Ende der Rolle und dessen entsprechende Stopfbuchseneinheit sind in ein gemeinsames Gehäuse eingekapselt. Spülgas wird aus einer Fernguelle durch Rohre in das Gehäuse geleitet, so dass es durch die Stopfbuchse und durch die Öffnung beziehungsweise durch die Lücke zwischen der Ofenwand und dem Schaft der Rolle hindurch in die Ofenkammer fließen kann.

Die japanische Offenlegungsschrift JP-A 06 185 876 offenbart eine Vorrichtung zur Abdichtung eines Vakuumofens. Eine Rolle führt durch die Ofenwand nach außen, wo sie in einer gekapselten Wälzlagereinheit gelagert wird. Zur ofenabgewandten Seite hin befinden sich ein inneres und ein äußeres Dichtungselement. Zwischen diese Dichtungselemente wird ein Gas eingeleitet, das an dem inneren Dichtungselement vorbei und durch die Wälzlagereinheit in den Ofen strömen kann.

Die japanische Offenlegungsschrift JP-A 07 090 352 schließlich offenbart einen Dichtungsmechanismus, durch den die Leckage von heißen Ofengasen aus einem Wärmebehandlungsofen verhindert werden kann. Der Wärmebehandlungsofen weist Rollen auf, die durch das Ofengehäuse nach außen geführt sind. Die Durchführungen weisen innenliegende Dichtungen zum Ofeninnenraum hin und zur Außenseite hin liegende äußere Dichtungen auf. Zwischen innerer und äußerer Dichtung befindet sich jeweils ein Gehäuse, in das Luft eingeleitet wird.

Eine Aufgabe der Erfindung ist es daher, einen Rollenherdofen zum Erwärmen von Werkstücken bereitzustellen, bei dem die Lebensdauer der Wälzlager für die Rollen des Rollenförderers erhöht wird.

Eine weitere Aufgabe der Erfindung ist es, ein Verfahren zum Erwärmen von Werkstücken bereitzustellen, bei dem ein solcher Rollenherdofen eingesetzt werden kann. Diese Aufgabe wird gelöst durch einen Rollenherdofen mit den Merkmalen des unabhängigen Anspruchs 1. Vorteilhafte Weiterbildungen des Rollenherdofens ergeben sich aus den Unteransprüchen 2 bis 7. Die Aufgabe wird ferner durch ein Verfahren nach Anspruch 8 gelöst.

Die Erfindung sieht einen Rollenherdofen zum Erwärmen von Werkstücken vor, der mindestens eine Ofenkammer zur Aufnahme der Werkstücke mit mindestens zwei seitlichen Ofenwänden umfasst. Innerhalb der Ofenkammer ist wenigstens eine Transportrolle zum Transport der Werkstücke durch die Ofenkammer angeordnet und die Ofenwände weisen jeweils wenigstens eine Öffnung auf, durch welche die Transportrolle hindurchführbar ist. An einer Außenseite der Ofenwände ist jeweils mindestens eine Wälzlagereinheit angeordnet, die dazu ausgebildet ist, die Transportrolle außerhalb der Ofenkammer aufzunehmen und zu lagern. Der Rollenherdofen weist ein Spülsystem für die Wälzlagereinheit auf, das dazu ausgebildet ist, ein Spülgas durch die Wälzlagereinheit zu leiten, wobei das Spülgas von einer von der Ofenwand abgewandten Seite der Wälzlagereinehit durch die Wälzlagereinheit und durch die Öffnung der Ofenwand hindurch in die Ofenkammer des Rollenherdofens leitbar ist. Dabei ist das Spülgas mit einem Druck bereitstellbar, der größer ist als ein Innendruck in der Ofenkammer des Rollenherdofens.

Die Zuführung eines Spülgases aus einem Spülsystem und die Durchleitung dieses Spülgases durch die Wälzlagereinheit haben den Vorteil, dass kein heißes Ofengas aus der Ofenkammer durch die Wälzlagereinheit hindurch strömen kann. Mit der Bereitstellung des Ofengases mit einem Druck, der größer ist als der Innendruck, der in der Ofenkammer herrscht, wird eine in die Ofenkammer gerichtete Spülgasströmung erzeugt, die verhindert, dass heißes und verunreinigtes Ofengas aus der Ofenkammer durch die Wälzlagereinheit strömen kann. Dadurch wird einerseits verhindert, dass sich das Schmiermittel der Wälzlagereinheit durch heißes Ofengas auflöst. Andererseits werden keine Verunreinigungspartikel aus der Ofenkammer in die Wälzlagereinheit eingetragen, die das Schmiermittel zerstören könnten. Da somit Zusammensetzung und Menge des Schmiermittels über einen längeren Zeitraum erhalten bleiben, werden dadurch ein niedrigerer Verschleiß und damit verbunden eine wesentlich längere Lebensdauer der Wälzlagereinheit erreicht, sodass die Wälzlagereinheit länger verwendet werden kann. Bedingt durch den niedrigeren Verschleiß erhöhen sich entsprechend die Austauschintervalle für die Wälzlagereinheit, sodass sich daraus auch ein ökonomischer Vorteil für einen derartigen Rollenherdofen aufgrund einer Kostenreduzierung ergibt.

Das Spülsystem des Rollenherdofens umfasst ein Rohrleitungssystem zum Zuführen des Spülgases zur Wälzlagereinheit, wobei das Rohrleitungssystem an der Außenseite der Ofenwand des Rollenherdofens angeordnet ist. Da die Temperatur an der Außenseite der Ofenwand der Ofenkammer nur etwa 100°C beträgt, ist mit einer Anordnung des Rohrleitungssystems für das Spülsystem an der Außenseite der Ofenwand sichergestellt, dass sich das Spülgas nicht zu sehr erwärmt, bevor es der Wälzlagereinheit zugeführt wird. Dies führt wiederum dazu, dass sich das Schmiermittel der Wälzlagereinheit nicht auflöst.

Außerdem besteht damit auch die Möglichkeit, konstruktionsbedingt möglicherweise bereits vorhandene Vorrichtungen des Rollenherdofens als Rohrleitungssystem zu verwenden. In einer Weiterbildung des Rollenherdofens ist deshalb vorgesehen, dass ein Stabilisator der Ofenwand als Rohrleitungssystem verwendbar ist. Dadurch müssen keine zusätzlichen Rohrleitungssysteme für die Zuführung des Spülgases zur Wälzlagereinheit installiert werden. Für die Installation des Rohrleitungssystems sind somit weder zusätzliche Aufwendungen für Material noch für die Montage erforderlich.

Weiterhin ist der Rollenherdofen dadurch gekennzeichnet, dass die Wälzlagereinheit ein inneres Wälzlager umfasst, neben dem ein äußeres Wälzlager so angeordnet ist, dass die beiden Wälzlager ein Wälzlagerpaar bilden, wobei das innere Wälzlager näher an der Außenseite der Ofenwand des Rollenherdofens angeordnet ist als das äußere Wälzlager, und dass zwischen den beiden Wälzlagern des Wälzlagerpaares ein Hohlraum ausbildet wird.

Bei dem Rollenherdofen ist ein Dichtelement an einer Seite des äußeren Wälzlagers angeordnet, die zu dem zwischen den beiden Wälzlagern ausgebildeten Hohlraum ausgerichtet ist.

In einer Weiterbildung des Rollenherdofens ist das Rohrleitungssystem des Spülsystems so mit dem Hohlraum verbunden, dass das Spülgas in den Hohlraum eingeleitet werden kann, wobei das Spülgas dann aus diesem Hohlraum durch das innere Wälzlager und durch die Öffnung der Ofenwand hindurch in die Ofenkammer des Rollenherdofens leitbar ist.

Durch die Ausbildung des Hohlraumes zwischen den beiden Wälzlagern des Wälzlagerpaares und die Verbindung des Hohlraumes mit dem Rohrleitungssystem für die Zuführung des Spülgases wird erreicht, dass das innere Wälzlager, das näher an der Außenseite der Ofenwand angeordnet ist und das somit austretendem Ofengas stärker ausgesetzt wäre, unmittelbar von dem Spülgas angeströmt und entsprechend durchströmt werden kann. Dieser Effekt wird noch dadurch verstärkt, dass das Dichtelement das äußere Wälzlager gegen das Spülgas im Hohlraum abdichtet.

Eine Ausgestaltung des Rollenherdofens sieht vor, dass das Spülsystem einen Strömungsmesser aufweist, wobei der Strömungsmesser dazu ausgebildet ist, eine Strömungsgeschwindigkeit des Spülgases zu messen, mit der das Spülgas durch die Wälzlagereinheit und durch die Öffnung der Ofenwand hindurch in die Ofenkammer des Rollenherdofens leitbar ist.

Die Verwendung eines Strömungsmessers ermöglicht eine Kontrolle der Strömungsgeschwindigkeit und damit des Drucks des Spülgases im Spülsystem. Dies gestaltet sich insofern als vorteilhaft, da bei einer zu niedrigen Strömungsgeschwindigkeit das Spülgas von aus der Ofenkammer austretendem Ofengas verdrängt werden könnte, sodass die Wälzlagereinheit nicht von Spülgas sondern von heißem Ofengas durchströmt werde würde. Eine zu hohe Strömungsgeschwindigkeit wiederum könnte zu einer Schädigung der Wälzlagereinheit durch durchströmendes Spülgas führen.

Eine Weiterbildung des Rollenherdofens sieht vor, dass das Spülsystem ein Steuerungselement aufweist, wobei das Steuerungselement dazu ausgebildet ist, die Strömungsgeschwindigkeit des Spülgases zu steuern. Über die Einstellmöglichkeiten mittels des Steuerungselementes kann somit auf Änderungen der atmosphärischen Bedingungen in der Ofenkammer entsprechend reagiert werden.

In einer Ausgestaltung des Rollenherdofens ist als Spülgas ein Schutzgas verwendbar. In einer anderen Ausgestaltung des Rollenherdofens ist als Spülgas Luft verwendbar, die gereinigt und/oder getrocknet sein kann.

Mit der Verwendung unterschiedlicher Gase als Spülgas kann das Spülsystem variabel an die Anforderungen verschiedener Rollenherdöfen, wie beispielsweise Öfen mit Schutzgasatmosphäre oder Öfen mit einem Luftstrom, angepasst werden. Damit wird erreicht, dass der erfindungsgemäße Rollenherdofen für unterschiedliche Anwendungsgebiete zur Verfügung steht.

Die Erfindung sieht ferner ein Verfahren zum Erwärmen von Werkstücken in einem Rollenherdofen vor. Dabei umfasst der Rollenherdofen mindestens eine Ofenkammer zur Aufnahme der Werkstücke mit mindestens zwei seitlichen Ofenwänden und innerhalb der Ofenkammer ist wenigstens eine Transportrolle zum Transport der Werkstücke durch die Ofenkammer angeordnet. Die Ofenwände weisen jeweils wenigstens eine Öffnung auf, durch welche die Transportrolle hindurch geführt ist und an einer Außenseite der Ofenwände ist jeweils mindestens eine Wälzlagereinheit angeordnet, die die Transportrolle außerhalb der Ofenkammer aufnimmt und lagert. In dem Verfahren wird mittels eines Spülsystems ein Spülgas durch die Wälzlagereinheit hindurch geleitet, wobei das Spülgas von einer von der Ofenwand abgewandten Seite der Wälzlagereinheit durch die Wälzlagereinheit und durch die Ofenwand hindurch in die Ofenkammer des Rollenherdofens geleitet wird. Dabei wird das Spülgas mit einem Druck bereitgestellt, der größer ist als der Innendruck in der Ofenkammer des Rollenherdofens.

Der Druck des Spülgases gewährleistet, dass das Spülgas die Wälzlagereinheit durchströmen kann und dass dabei das Ofengas aus der Wälzlagereinheit und aus der Öffnung in der Ofenwand verdrängt wird.

Die zuvor genannten und weitere Vorteile, Besonderheiten und zweckmäßige Weiterbildungen der Erfindung werden auch anhand der Ausführungsbeispiele deutlich, die nachfolgend unter Bezugnahme auf die Figuren beschrieben werden.

Von den Figuren zeigt:
- Fig. 1a: einen Längsschnitt durch einen Rollenherdofen,
- Fig. 1b: einen Querschnitt des Rollenherdofens,
- Fig. 2a: ein Kugellager,
- Fig. 2b: eine Schnittdarstellung B-B des Kugellagers und
- Fig. 3: eine Schnittdarstellung eines Bildausschnitts A aus Fig. 1b mit einem Spülsystem.

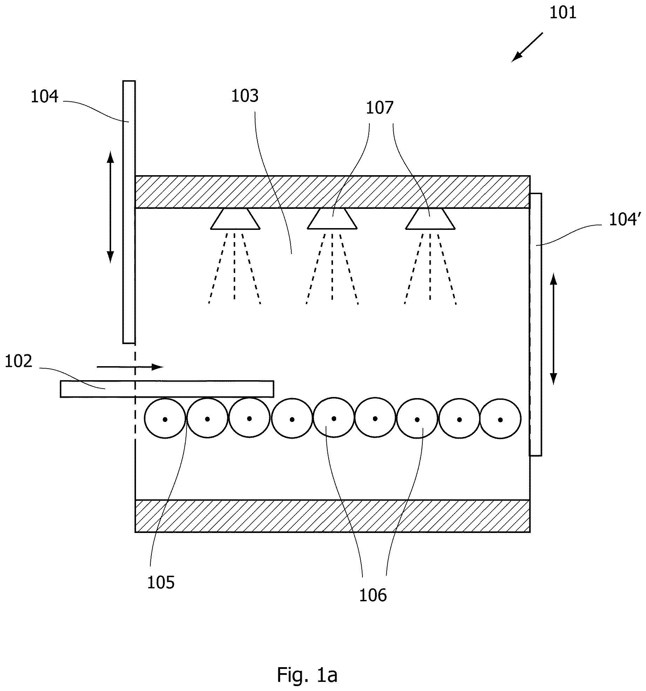

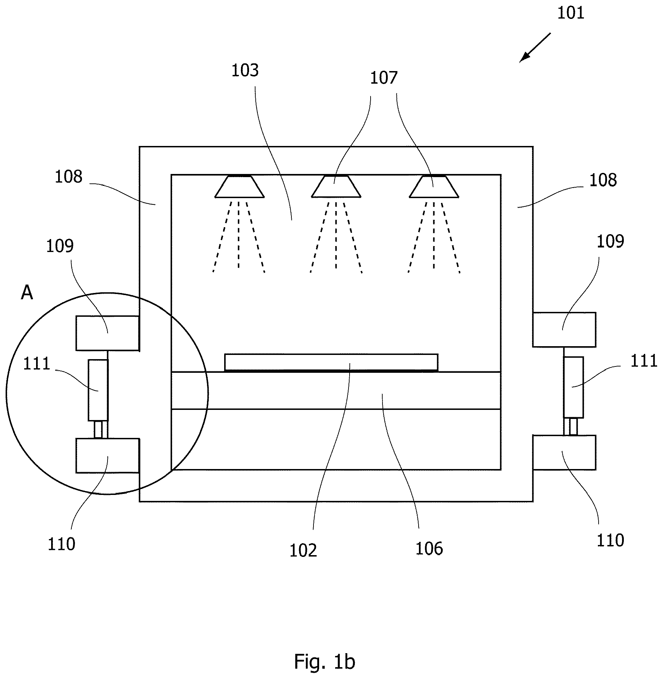

Fig. 1 zeigt schematisch als Beispiel einen Längsschnitt durch einen Rollenherdofen 101 zum Erwärmen eines Werkstücks 102. Der Rollenherdofen 101 umfasst eine längs gestreckte Ofenkammer 103, die vorn und hinten mit jeweils einer höhenverstellbaren Kammertür 104, 104' verschlossen werden kann. Vor einer Zuführung bzw. vor einer der Entnahme des Werkstückes 102 aus der Ofenkammer 103 öffnen sich die Kammertüren 104, 104' und nach Zuführung bzw. Entnahme schließen sich die Kammertüren 104, 104' entsprechend wieder. Diese Kammertüren 104 und 104' können beispielsweise als Ofenschieber ausgebildet sein. Obwohl die Ausgestaltung des Rollenherdofens 101 mit Kammertüren 104, 104' insbesondere auch in Bezug auf die Wärmeverteilung im Rollenherdofen 101 als vorteilhaft erscheint, kann ein Rollenherdofen 101 bei Bedarf aber auch ohne Kammertüren 104, 104' betrieben werden.

Der Rollenherdofen 101 weist eine Transporteinrichtung für die Werkstücke 102 in Form eines Rollenförderers 105 auf, der innerhalb der Ofenkammer 103 angeordnet ist und der das Werkstück 102 zum Erwärmen in Pfeilrichtung durch den Rollenherdofen 101 transportiert. Der Rollenförderer 105 wird dabei aus mehreren hintereinander angeordneten Transportrollen 106 gebildet, die entweder einzeln oder auch gruppenweise angetrieben werden.

Obwohl in Fig. 1a beispielhaft nur ein Werkstück 102 dargestellt ist, können mit dem Rollenförderer 105 auch mehrere Werkstücke 102 gleichzeitig zum Erwärmen durch den Rollenherdofen 101 transportiert werden, wobei mehrere Werkstücke 102 hintereinander und/oder nebeneinander transportiert werden können.

In der Ofenkammer 103 sind geeignete Heizelemente 107 angeordnet, mit denen die Werkstücke 102 beim Durchlaufen des Rollenherdofens 101 erwärmt werden können. Derartige Heizelemente 107 sind aus dem Stand der Technik bekannt und werden nicht im Einzelnen erläutert. Auch alle weiteren erforderlichen Komponenten zum Betrieb des Rollenherdofens 101 sind nicht Gegenstand der Erfindung und können vom Fachmann geeignet gewählt werden.

In Fig. 1b ist beispielhaft ein Querschnitt des Rollenherdofens 101 dargestellt. Aus Gründen der Übersichtlichkeit wurde hier die vordere Kammertür 104 der Ofenkammer 103 nicht dargestellt. Die Ofenkammer 103 des Rollenherdofens 101 weist auf beiden Seiten jeweils eine Ofenwand 108, 108' zur seitlichen Begrenzung der Ofenkammer 103 auf.

Jeweils an der Außenseite der beiden Ofenwände 108, 108' der Ofenkammer 103 ist ein Stabilisator 109 zur mechanischen Stabilisierung der Ofenkammer 103 angeordnet. Diese mechanische Stabilisierung ist erforderlich, da das Material der Ofenwände 108, 108' durch die teilweise sehr hohen Temperaturunterschiede zwischen dem Innenraum der Ofenkammer 103 und ihrer Umgebung großen thermischen Belastungen unterliegt, die zu Verformungen der Ofenwände 108, 108' führen können. Obwohl hier beispielhaft nur ein Stabilisator 109 dargestellt ist, können aber auch mehrere Stabilisatoren 109 zur Erhöhung der Stabilisierungswirkung an den Außenseiten der beiden Ofenwände 108, 108' angeordnet sein.

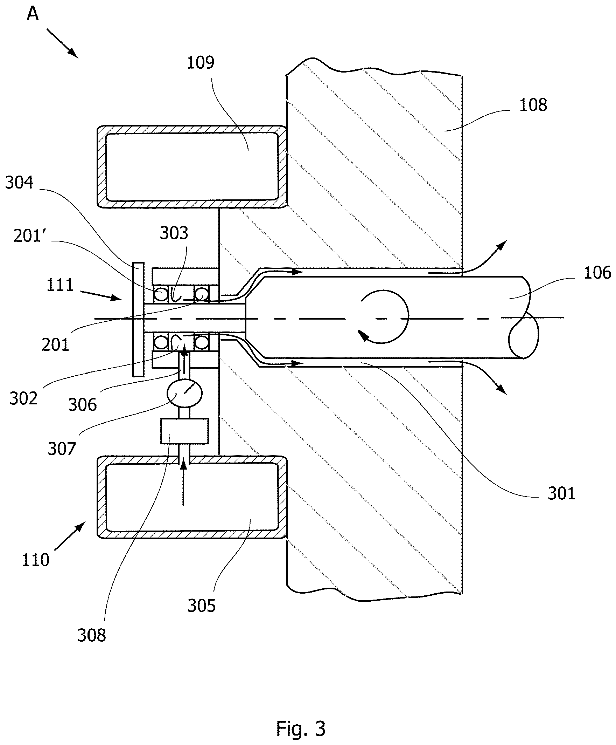

Weiterhin befindet sich jeweils an der Außenseite der beiden Ofenwände 108, 108' ein Spülsystem 110 zum Spülen der ebenfalls dort angeordneten Wälzlagereinheit 111. Aufbau und Funktionsweise des Spülsystems 110 für die Wälzlagereinheit 111 werden in einem nachfolgenden Abschnitt zu Fig. 3 anhand einer vergrößerten Schnittdarstellung des Bildausschnitts A aus Fig. 1b näher erläutert.

Die Wälzlagereinheit 111 an der Außenseite der beiden Außenwände 108, 108' der Ofenkammer 103 dient jeweils der Aufnahme und Lagerung eines der beiden Enden der einzelnen Rollen 106 des Rollenförderers 105. Damit die Rollen 106 in den Wälzlagereinheiten 111 außerhalb der Ofenkammer 103 gelagert werden können, sind in den Ofenwänden 108, 108' hier nicht dargestellte Öffnungen ausgebildet, durch die die einzelnen Rollen 106 jeweils aus der Ofenkammer 103 heraus nach außen in die zugehörige Wälzlagereinheit 111 geführt werden.

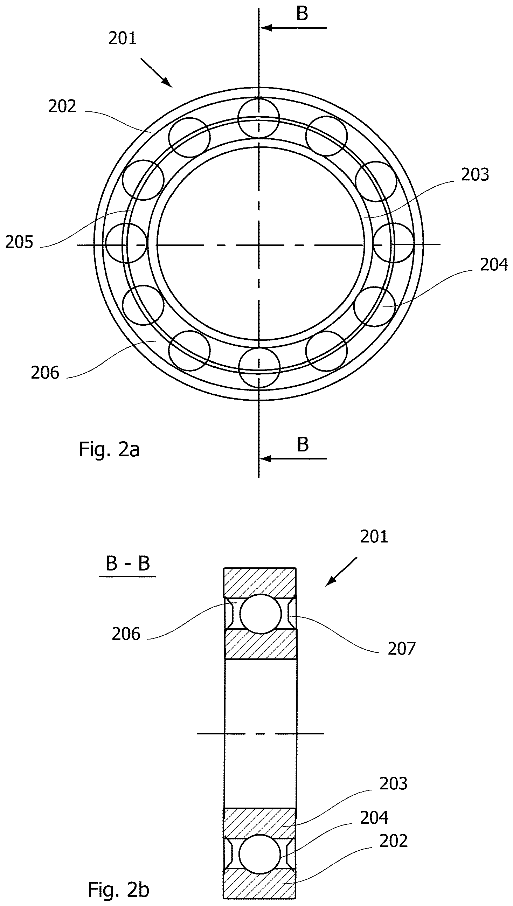

Ein wesentlicher Bestandteil der Wälzlagereinheiten 111 zur beidseitigen Aufnahme und Lagerung der einzelnen Rollen 106 sind Wälzlager 201. In Fig. 2a ist schematisch das Beispiel eines solchen Wälzlagers 201 für eine Wälzlagereinheit 111 dargestellt. Das Wälzlager 201 ist hier als Kugellager ausgebildet, das einen Außenring 202 und einen Innenring 203 aufweist, zwischen denen sich als Wälzkörper einzelne Kugeln 204 befinden, die zwischen dem Außenring 202 und dem Innenring 203 Rollbewegungen ausführen können. Die Kugeln 204 werden dabei üblicherweise von einem Kugelkäfig 205 zwischen dem Außenring 202 und dem Innenring 203 positioniert und geführt. Es gibt aber auch Ausführungen von Kugellagern 201 ohne Kugelkäfig 205. Zwischen den einzelnen Kugeln 204 sind Hohlräume ausgebildet, die mit einem Schmierstoff 206 gefüllt sind.

Dieser Schmierstoff 206 hat insbesondere die Aufgabe, die während der Rollbewegungen der Kugeln 204 auftretende Reibung zwischen den Kugeln 204 und den beiden Ringen 202, 203 zu verringern und dadurch die Verlustleistung und den Verschleiß des Kugellagers 201 zu minimieren. Als Schmierstoff 206 geeignet sind dabei alle bekannten Schmierstoffe und -fette, die in Wälzlagern üblicherweise zur Anwendung kommen.

Fig. 2b zeigt schematisch eine Schnittdarstellung B-B durch das Kugellager 201. Das Kugellager 201 weist hier zusätzlich auf beiden Seiten jeweils noch eine Dichtscheibe 207 auf, die das Innere des Kugellagers vor dem Eindringen von Schmutz oder Feuchtigkeit schützt und gleichzeitig das Austreten von Schmierstoff 206 verhindert. Insbesondere in Abhängigkeit von der geplanten Anwendung der Kugellager 201 und den sich daraus ergebenden Anforderungen beispielsweise an Spitzwasser- oder Staubschutz sind Dichtscheiben 207 aus unterschiedlichen Materialien bekannt, beispielsweise aus Kautschuk für einen Spritzwasserschutz oder aus Stahlblech für einen Staubschutz. Auch verschiedene Kunststoffe oder Verbundmaterialien können für die Dichtscheiben 207 verwendet werden.

Das Material der Dichtscheiben 207 in Verbindung mit der Anzahl der Dichtscheiben 207 bestimmt den Typ des Kugellagers 201. Neben weiteren Kombinationen werden Kugellager insbesondere in folgende Typen eingeteilt:
* Kugellager mit einer Dichtscheibe als Spritzwasserschutz - R-Typ
* Kugellager mit zwei Dichtscheiben als Spritzwasserschutz - RR- oder 2R-Typ
* Kugellager mit einer Dichtscheibe als Staubschutz - Z-Typ
* Kugellager mit zwei Dichtscheiben als Staubschutz - ZZ- oder 2Z-Typ

Die Kugellager 201 der Wälzlagereinheiten 111 dienen einerseits der Aufnahme und Fixierung der Rollen 106 des Rollenförderers 105 außerhalb der Ofenkammer 103 und ermöglichen andererseits eine Drehbewegung der Rollen 106 innerhalb der Ofenkammer 103, die wiederum für den Transport des Werkstücks 102 durch den Rollenherdofen 101 verantwortlich zeichnet.

Obwohl die Verwendung von Kugellagern 201 für die Lagerung der Rollen 106 vorteilhaft erscheint, können auch andere Wälzlager, wie beispielsweise Zylinderrollenlager, Kegelrollenlager oder andere Wälzlager verwendet werden.

Bedingt durch die Anordnung der Wälzlagereinheiten 111 an den Außenseiten der Ofenwände 108, 108' sind die thermischen und mechanischen Belastungen der Kugellager 201 sehr hoch. Dies wiederum verursacht einen hohen Verschleiß der Kugellager 201 und mit zunehmendem Verschleiß der Kugellager 201 kann sich das Drehverhalten der Rollen 106 von leichtgängig über schwergängig bis hin zu einer möglichen Blockade der Rollen 106 verändern. Eine Reduzierung des Verschleißes wird durch den Einsatz des Spülsystems 110 erreicht.

Fig. 3 zeigt schematisch eine vergrößerte Schnittdarstellung des Bildausschnitts A aus Fig. 1b mit einem Teil einer Ofenwand 108 und einem Teil einer Rolle 106 sowie mit einem Stabilisator 109, einem Spülsystem 110 und einer Wälzlagereinheit 111.

Da die Lagerung der Rolle 106 außerhalb der Ofenkammer 103 erfolgt, ist in der Ofenwand 108 eine Öffnung 301 ausgebildet, durch die die Rolle 106 aus der Ofenkammer 103 heraus nach außen in die zugehörige Wälzlagereinheit 111 geführt wird. Die Lagerung der Rolle 106 in der Wälzlagereinheit 111 wird über ein Kugellagerpaar realisiert, das aus einem inneren Kugellager 201 und einem äußeren Kugellager 201' gebildet wird. Dabei ist das innere Kugellager 201 des Kugellagerpaares näher an der Ofenwand 108 angeordnet als das äußere Kugellager 201' und beide Kugellager 201 und 201' sind so nebeneinander angeordnet, dass zwischen den beiden Kugellagern 201 und 201' des Kugellagerpaares ein Hohlraum 302 ausgebildet wird. Beide Kugellager 201 und 201' sind vom ZZ-Typ, weisen also je zwei Dichtscheiben 207 als Staubschutz auf.

Im Inneren des Hohlraumes 302 an der Seite des äußeren Kugellagers 201', die zum Hohlraum 302 ausgerichtet ist, ist ein Dichtelement 303 angeordnet. Da die Dichtscheiben 207 des äußeren Kugellagers 201' nur als Staubschutz ausgebildet sind, ist es Aufgabe dieses Dichtelementes 303, den Hohlraum 302 so abzudichten, dass kein Spülgas aus dem Hohlraum 302 in das äußere Kugellager 201' eindringen kann. Dafür kann das Dichtelement 303 als Wellendichtring, beispielsweise als Radialwellendichtring, ausgebildet sein.

Die Sicherung des äußeren Kugellagers 201' auf der Rolle 106 erfolgt über einen Sicherungsring 304, der am Ende der Rolle 106 montiert wird. Bei diesem Sicherungsring 304 handelt es sich beispielsweise um ein entsprechendes Normteil nach DIN 471. Es können aber auch andere Sicherungen für das äußere Kugellager 201' verwendet werden.

Mit der Wälzlagereinheit 111 ist das Spülsystem 110 verbunden. Ein Bestandteil dieses Spülsystems 110 ist ein Rohrleitungssystem 305, das unterhalb der Wälzlagereinheit 111 angeordnet ist und das über eine Zuleitung 306 an den Hohlraum 302 des Wälzlagerpaares der Wälzlagereinheit 111 angebunden ist. In die Zuleitung 306 integriert sind ein Strömungsmesser 307 sowie ein Steuerungselement 308.

Als Rohrleitungssystem 305 für das Spülgas können an der Außenseite der Ofenwand 108 beispielsweise separate Gasleitungen installiert werden. Es ist aber auch möglich, konstruktionsbedingt bereits vorhandene Rohre oder Gehäuse des Rollenherdofens 101, die entsprechend den bereits beschriebenen Stabilisatoren 109 als weitere Stabilisatoren 109 an der Außenseite der Ofenwand 108 angeordnet sind, als Rohrleitungssystem 305 für das Spülgas zu nutzen.

Die Zuleitung 306 kann beispielsweise als flexibler Gummi- oder Silikonschlauch ausgeführt werden. Es können aber auch feste Leitungen beispielsweise aus Kunststoff oder Metall installiert werden. Bei der Materialauswahl für die Zuleitung 306 sind vor allem die Anforderungen des verwendeten Spülgases hinsichtlich einer chemischen Beständigkeit sowie die geplanten Druckverhältnisse zu berücksichtigen.

Für eine leichte und einfache Montage kann die Zuleitung 306 beispielsweise jeweils über Steckverbindungen mit dem Hohlraum 302 und dem Rohrleitungssystem 305 verbunden werden. Es sind aber auch andere Verbindungsarten möglich.

Zum Spülen des inneren Kugellagers 201 wird in das Rohrleitungssystem 305 ein konditioniertes Spülgas eingespeist. In Abhängigkeit von der Art des Rollenherdofens 101 kann es sich bei dem Spülgas beispielsweise um ein Schutzgas für Öfen mit Schutzgasatmosphäre oder auch um einen Luftstrom für Öfen mit Luft handeln. Andere Gase sind aber ebenfalls als Spülgas verwendbar.

Das Spülgas wird im Rohrleitungssystem 305 mit einem Druck bereitgestellt, der einen Gasstrom in einer Größenordnung von ungefähr 10l pro Stunde erzeugt. Die Strömungsgeschwindigkeit des Spülgases kann am Strömungsmesser 307 entsprechend abgelesen und überwacht werden. Eine Regulierung des Gasstromes erfolgt mittels des Steuerungselementes 308, bei dem es sich im einfachsten Fall um eine Drossel oder um ein Einstellventil zur manuellen Einstellung des Gasstromes handelt. Ebenfalls als Steuerungselement 308 verwendbar sind einstellbare Düsen oder Blenden, mit denen die Strömungsgeschwindigkeit des Spülgases beeinflusst werden kann, indem beispielsweise Form und Größe der Düsen- oder Blendenöffnungen variiert werden. Weiterhin besteht die Möglichkeit, ein komplexes Steuerungselement 308 zu installieren, über das eine automatische Steuerung des Gasstromes möglich ist.

Durch den anliegenden Druck wird das Spülgas aus dem Rohrleitungssystem 305 über die Zuleitung 306 in den Hohlraum 302 des Kugellagerpaares eingeleitet. Dabei wird auch das innere Kugellager 201 entsprechend angeströmt. Da die Dichtscheiben 207 des inneren Kugellagers 201 lediglich einen Staub- und keinen Gasschutz realisieren und da das innere Kugellager 201 bedingt durch seinen Aufbau an unterschiedlichsten Stellen kleine Schlitze und Spalte aufweist, kann das Spülgas das angeströmte innere Kugellager 201 auch durchströmen. Dafür dringt das Spülgas auf der Seite des inneren Kugellagers 201, die zum Hohlraum 302 ausgerichtet ist, durch die Schlitze und Spalte in das innere Kugellager 201 ein und tritt auf der Seite des inneren Kugellagers 201, die zur Ofenwand 108 ausgerichtet ist, wieder aus. Ein gleichzeitiges Durchströmen des äußeren Kugellagers 201' ist dabei nicht möglich, da das äußere Kugellager 201' bedingt durch das Dichtelement 303 vom Spülgas nicht angeströmt werden kann.

Nach dem Durchströmen des inneren Kugellagers 201 strömt das Spülgas dann weiter durch die Öffnung 301 in der Ofenwand 108 in das Innere der Ofenkammer 103. Der gesamte Strömungsweg des Spülgases aus dem Rohrleitungssystem 305 durch die Zuleitung 306 in den Hohlraum 302 und dann weiter durch das innere Kugellager 201 und durch die Öffnung 301 in der Ofenwand 108 in die Ofenkammer 103 ist entsprechend durch Pfeile gekennzeichnet.

Über den eingestellten Druck des Spülgases kann zuverlässig und effektiv verhindert werden, dass Ofengas aus der Ofenkammer 103 durch die Öffnung 301 und durch das innere Kugellager 201 strömt. Dadurch wird verhindert, dass sich der Schmierstoff 206 des inneren Kugellagers 201, bedingt durch heißes Ofengas, auflöst. Weiterhin wird zuverlässig verhindert, dass die während der Drehung der Rollen 16 und der damit verbundenen Reibung an der keramischen Isolierwand des Rollenherdofens 101 entstehenden Partikel mit dem Ofengas in das innere Kugellager 201 eingetragen werden. Vielmehr wird das innere Kugellager 201 durch das Spülgas frei von Partikeln gehalten, da das Spülgas nicht verunreinigt ist. Zudem ist die Temperatur des Spülgases wesentlich niedriger als die Temperatur des Ofengases, da es an der Außenseite des Rollenherdofens 101 an die Wälzlagereinheit 111 herangeführt wird, sodass sich der Schmierstoff 206 während des Durchströmens auch nicht auflöst.

Da ein Rollenförderer 105 aus vielen, hintereinander angeordneten Rollen 106 besteht, weisen die beiden Ofenwände 108, 108' eine entsprechende Anzahl von Öffnungen 301 auf, durch die die Rollen 106 aus der Ofenkammer 103 heraus in die entsprechend zugehörigen Wälzlagereinheiten 111 geführt werden. Jede dieser Wälzlagereinheiten 111 ist gekennzeichnet durch ein Wälzlagerpaar mit einem inneren Wälzlager 201 und einem äußeren Wälzlager 201', zwischen denen jeweils ein Hohlraum 302 ausgebildet wird, von denen wiederum jeder über eine separate Zuleitung 306 mit dem Rohrleitungssystem 305 verbunden ist. In jede separate Zuleitung 306 sind dabei sowohl ein Strömungsmesser 307 als auch ein Steuerungselement 308 integriert.

Das Rohrleitungssystem 305 benötigt zwar nur einen Anschluss für die Zufuhr des Spülgases in das Rohrleitungssystem 305, auch wenn mehrere Anschlüsse dafür vorgesehen werden können, es versorgt aber eine Vielzahl von inneren Kugellagern 201 mit Spülgas und gewährleistet damit, dass der Verschleiß aller inneren Kugellager 201 entsprechend reduziert wird.

Zur Überwachung der Dichtheit der einzelnen Wälzlagereinheiten 111 und damit zur Überwachung der Funktion des Spülsystems 110 kann im Inneren der Hohlräume 302 der einzelnen Wälzlagereinheiten 111 ein weiteres Dichtelement 303 so angeordnet werden, dass eine Kammer ausgebildet wird. Mit einer an diese Kammer angeschlossenen Drucküberwachung besteht dann die Möglichkeit, den Druck in der Kammer zu überwachen und bei einem registrierten Druckabfall in dieser Kammer, die betroffene Wälzlagereinheit 111 entsprechend auszutauschen.

### Bezugszeichenliste:

- 101: Rollenherdofen
- 102: Werkstück
- 103: Ofenkammer
- 104, 104': Kammertür
- 105: Rollenförderer
- 106: Transportrolle, Rolle
- 107: Heizelement
- 108, 108': Ofenwand
- 109: Stabilisator
- 110: Spülsystem
- 111: Wälzlagereinheit

- 201: Wälzlager/Kugellager, inneres Wälzlager/Kugellager
- 201': Wälzlager/Kugellager, äußeres Wälzlager/Kugellager
- 202: Außenring
- 203: Innenring
- 204: Kugel
- 205: Kugelkäfig
- 206: Schmierstoff
- 207: Dichtscheibe

- 301: Öffnung
- 302: Hohlraum
- 303: Dichtelement
- 304: Sicherungsring
- 305: Rohrleitungssystem
- 306: Zuleitung
- 307: Strömungsmesser
- 308: Steuerungselement

## Patentansprüche

1. Rollenherdofen (101) zum Erwärmen von Werkstücken (102), umfassend mindestens eine Ofenkammer (103) zur Aufnahme der Werkstücke (102) mit mindestens zwei seitlichen Ofenwänden (108, 108'), wobei innerhalb der Ofenkammer (103) wenigstens eine Transportrolle (106) zum Transport der Werkstücke (102) durch die Ofenkammer (103) angeordnet ist und wobei die Ofenwände (108, 108') jeweils wenigstens eine Öffnung (301) aufweisen, durch welche die Transportrolle (106) hindurchführbar ist und wobei an einer Außenseite der Ofenwände (108, 108') jeweils mindestens eine Wälzlagereinheit (111) angeordnet ist, die dazu ausgebildet ist, die Transportrolle (106) außerhalb der Ofenkammer (103) aufzunehmen und zu lagern,
**dadurch gekennzeichnet,**
**dass** der Rollenherdofen (101) ein Spülsystem (110) für die Wälzlagereinheit (111) aufweist, das dazu ausgebildet ist, ein Spülgas durch die Wälzlagereinheit (111) zu leiten, wobei das Spülgas von einer von der Ofenwand (108, 108') abgewandten Seite der Wälzlagereinheit (111) durch die Wälzlagereinheit (111) und durch die Öffnung (301) der Ofenwand (108, 108') hindurch in die Ofenkammer (103) des Rollenherdofens (101) leitbar ist und wobei das Spülgas mit einem Druck bereitstellbar ist, der größer ist als ein Innendruck in der Ofenkammer (103) des Rollenherdofens (101), wobei das Spülsystem (110) ein Rohrleitungssystem (305) zum Zuführen des Spülgases zur Wälzlagereinheit (111) umfasst und das Rohrleitungssystem (305) an der Außenseite der Ofenwand (108, 108') des Rollenherdofens (101) angeordnet ist und wobei die Wälzlagereinheit (111) ein inneres Wälzlager (201) umfasst, neben dem ein äußeres Wälzlager (201') so angeordnet ist, dass die beiden Wälzlager (201, 201') ein Wälzlagerpaar bilden, und wobei das innere Wälzlager (201) näher an der Außenseite der Ofenwand (108, 108') des Rollenherdofens (101) angeordnet ist als das äußere Wälzlager (201'), und dass zwischen den beiden Wälzlagern (201, 201') des Wälzlagerpaares ein Hohlraum (302) ausbildet wird, wobeiein Dichtelement (303) an einer Seite des äußeren Wälzlagers (201') angeordnet ist, die zu dem zwischen den beiden Wälzlagern (201, 201') des Wälzlagerpaares ausgebildeten Hohlraum (302) ausgerichtet ist.

2. Rollenherdofen (101) nach Anspruch 1,
**dadurch gekennzeichnet,**
**dass** ein Stabilisator (109) der Ofenwand (108, 108') als Rohrleitungssystem (305) verwendbar ist.

3. Rollenherdofen (101) nach einem der vorherigen Ansprüche,
**dadurch gekennzeichnet,**
**dass** das Rohrleitungssystem (305) des Spülsystems (110) so mit dem Hohlraum (302) verbunden ist, dass das Spülgas in den Hohlraum (302) eingeleitet werden kann, wobei das Spülgas dann aus diesem Hohlraum (302) durch das innere Wälzlager (201) und durch die Öffnung (301) der Ofenwand (108, 108') hindurch in die Ofenkammer (103) des Rollenherdofens (101) leitbar ist.

4. Rollenherdofen (101) nach einem der vorherigen Ansprüche,
**dadurch gekennzeichnet,**
**dass** das Spülsystem (110) einen Strömungsmesser (307) aufweist, wobei der Strömungsmesser (307) dazu ausgebildet ist, eine Strömungsgeschwindigkeit des Spülgases zu messen, mit der das Spülgas durch das Wälzlager (201, 201') und durch die Öffnung (301) der Ofenwand (108, 108') hindurch in die Ofenkammer (103) des Rollenherdofens (101) leitbar ist.

5. Rollenherdofen (101) nach Anspruch 4,
**dadurch gekennzeichnet,**
**dass** das Spülsystem (110) ein Steuerungselement (308) aufweist, wobei das Steuerungselement (308) dazu ausgebildet ist, die Strömungsgeschwindigkeit des Spülgases zu steuern.

6. Rollenherdofen (101) nach einem der vorherigen Ansprüche,
**dadurch gekennzeichnet,**
**dass** als Spülgas ein Schutzgas verwendbar ist.

7. Rollenherdofen (101) nach einem der vorherigen Ansprüche,
**dadurch gekennzeichnet,**
**dass** als Spülgas Luft verwendbar ist, wobei diese Luft gereinigt und/oder getrocknet sein kann.

8. Verfahren zum Erwärmen von Werkstücken (102) in einem Rollenherdofen (101), wobei der Rollenherdofen (101) mindestens eine Ofenkammer (103) zur Aufnahme der Werkstücke (102) mit mindestens zwei seitlichen Ofenwänden (108, 108') umfasst und wobei innerhalb der Ofenkammer (103) wenigstens eine Transportrolle (106) zum Transport der Werkstücke (102) durch die Ofenkammer (103) angeordnet ist, wobei die Ofenwände (108, 108') jeweils wenigstens eine Öffnung (301) aufweisen, durch welche die Transportrolle (106) hindurch geführt ist und wobei an einer Außenseite der Ofenwände (108, 108') jeweils mindestens eine Wälzlagereinheit (111) angeordnet ist, die die Transportrolle (106) außerhalb der Ofenkammer (103) aufnimmt und lagert,
**dadurch gekennzeichnet,**
**dass** mittels eines Spülsystems (110) ein Spülgas durch die Wälzlagereinheit (111) hindurch geleitet wird, wobei das Spülgas von einer von der Ofenwand (108, 108') abgewandten Seite der Wälzlagereinheit (111) durch die Wälzlagereinheit (111) und durch die Ofenwand (108, 108') hindurch in die Ofenkammer (103) des Rollenherdofens (101) geleitet wird und wobei das Spülgas mit einem Druck bereitgestellt wird, der größer ist als ein Innendruck in der Ofenkammer (103) des Rollenherdofens (101), wobei das Spülsystem (110) ein Rohrleitungssystem (305) zum Zuführen des Spülgases zur Wälzlagereinheit (111) umfasst und das Rohrleitungssystem (305) an der Außenseite der Ofenwand (108, 108') des Rollenherdofens (101) angeordnet ist wobei die Wälzlagereinheit (111) ein inneres Wälzlager (201) umfasst, neben dem ein äußeres Wälzlager (201') so angeordnet ist, dass die beiden Wälzlager (201, 201') ein Wälzlagerpaar bilden, und wobei das innere Wälzlager (201) näher an der Außenseite der Ofenwand (108, 108') des Rollenherdofens (101) angeordnet ist als das äußere Wälzlager (201'), und dass zwischen den beiden Wälzlagern (201, 201') des Wälzlagerpaares ein Hohlraum (302) ausbildet wird, wobeiein Dichtelement (303) an einer Seite des äußeren Wälzlagers (201') angeordnet ist, die zu dem zwischen den beiden Wälzlagern (201, 201') des Wälzlagerpaares ausgebildeten Hohlraum (302) ausgerichtet ist.

## Claims

1. Roller hearth furnace (101) for heating workpieces (102), comprising at least one furnace chamber (103) for receiving the workpieces (102) that has at least two lateral furnace walls (108, 108'), wherein at least one transport roller (106) for transporting the workpieces (102) through the furnace chamber (103) is arranged within the furnace chamber (103), and wherein the furnace walls (108, 108') each have at least one opening (301) through which the transport roller (106) can be guided, and wherein at least one rolling bearing unit (111) is arranged on each outer side of the furnace walls (108, 108') and is designed to receive and bear the transport roller (106) outside the furnace chamber (103),
**characterized**
**in that** the roller hearth furnace (101) has a flushing system (110) for the rolling bearing unit (111) that is designed to channel a flushing gas through the rolling bearing unit (111), wherein the flushing gas can be channeled from a side of the rolling bearing unit (111) facing away from the furnace wall (108, 108') through the rolling bearing unit (111) and through the opening (301) of the furnace wall (108, 108') into the furnace chamber (103) of the roller hearth furnace (101), and wherein the flushing gas can be provided at a pressure which is greater than an internal pressure in the furnace chamber (103) of the roller hearth furnace (101), wherein the flushing system (110) comprises a pipeline system (305) for feeding the flushing gas to the rolling bearing unit (111), and the pipeline system (305) is arranged on the outer side of the furnace wall (108, 108') of the roller hearth furnace (101), and wherein the rolling bearing unit (111) comprises an inner rolling bearing (201) next to which an outer rolling bearing (201') is arranged in such a way that the two rolling bearings (201, 201') form a rolling bearing pair, and wherein the inner rolling bearing (201) is arranged closer to the outer side of the furnace wall (108, 108') of the roller hearth furnace (101) than the outer rolling bearing (201'), and in that a cavity (302) is formed between the two rolling bearings (201, 201') of the rolling bearing pair, wherein a sealing element (303) is arranged on a side of the outer rolling bearing (201') that is oriented with respect to the cavity (302) formed between the two rolling bearings (201, 201') of the rolling bearing pair.

2. Roller hearth furnace (101) according to Claim 1,
**characterized**
**in that** a stabilizer (109) of the furnace wall (108, 108') can be used as pipeline system (305).

3. Roller hearth furnace (101) according to one of the preceding claims,
**characterized**
**in that** the pipeline system (305) of the flushing system (110) is connected to the cavity (302) in such a way that the flushing gas can be introduced into the cavity (302), wherein the flushing gas can then be channelled out of this cavity (302) through the inner rolling bearing (201) and through the opening (301) of the furnace wall (108, 108') into the furnace chamber (103) of the roller hearth furnace (101).

4. Roller hearth furnace (101) according to one of the preceding claims,
**characterized**
**in that** the flushing system (110) has a flowmeter (307), wherein the flowmeter (307) is designed to measure a flow velocity of the flushing gas at which the flushing gas can be channelled through the rolling bearing (201, 201') and through the opening (301) of the furnace wall (108, 108') into the furnace chamber (103) of the roller hearth furnace (101) .

5. Roller hearth furnace (101) according to Claim 4,
**characterized**
**in that** the flushing system (110) has a control element (308), wherein the control element (308) is designed to control the flow velocity of the flushing gas.

6. Roller hearth furnace (101) according to one of the preceding claims,
**characterized**
**in that** a protective gas can be used as flushing gas.

7. Roller hearth furnace (101) according to one of the preceding claims,
**characterized**
**in that** air can be used as flushing gas, wherein this air can be cleaned and/or dried.

8. Method for heating workpieces (102) in a roller hearth furnace (101), wherein the roller hearth furnace (101) comprises at least one furnace chamber (103) for receiving the workpieces (102) that has at least two lateral furnace walls (108, 108'), and wherein at least one transport roller (106) for transporting the workpieces (102) through the furnace chamber (103) is arranged within the furnace chamber (103), wherein the furnace walls (108, 108') each have at least one opening (301) through which the transport roller (106) is guided, and wherein at least one rolling bearing unit (111) is arranged on each outer side of the furnace walls (108, 108') and receives and bears the transport roller (106) outside the furnace chamber (103),
**characterized**
**in that** a flushing gas is channelled through the rolling bearing unit (111) by means of a flushing system (110), wherein the flushing gas is channelled from a side of the rolling bearing unit (111) facing away from the furnace wall (108, 108') through the rolling bearing unit (111) and through the furnace wall (108, 108') into the furnace chamber (103) of the roller hearth furnace (101), and wherein the flushing gas is provided at a pressure which is greater than an internal pressure in the furnace chamber (103) of the roller hearth furnace (101), wherein the flushing system (110) comprises a pipeline system (305) for feeding the flushing gas to the rolling bearing unit (111), and the pipeline system (305) is arranged on the outer side of the furnace wall (108, 108') of the roller hearth furnace (101), wherein the rolling bearing unit (111) comprises an inner rolling bearing (201) next to which an outer rolling bearing (201') is arranged in such a way that the two rolling bearings (201, 201') form a rolling bearing pair, and wherein the inner rolling bearing (201) is arranged closer to the outer side of the furnace wall (108, 108') of the roller hearth furnace (101) than the outer rolling bearing (201'), and in that a cavity (302) is formed between the two rolling bearings (201, 201') of the rolling bearing pair, wherein a sealing element (303) is arranged on a side of the outer rolling bearing (201') that is oriented with respect to the cavity (302) formed between the two rolling bearings (201, 201') of the rolling bearing pair.

## Revendications

1. Four à rouleaux (101) pour chauffer des pièces (102), comprenant au moins une chambre de four (103) pour recevoir les pièces (102) avec au moins deux parois de four latérales (108, 108'), au moins un rouleau de transport (106) pour le transport des pièces (102) à travers la chambre de four (103) étant disposé à l'intérieur de la chambre de four (103) et les parois de four (108, 108') présentant chacune au moins une ouverture (301) à travers laquelle le rouleau de transport (106) peut être guidé et à chaque fois au moins une unité de paliers à roulement (111) étant disposée au niveau d'un côté extérieur des parois de four (108, 108'), laquelle est réalisée de manière à recevoir et supporter le rouleau de transport (106) à l'extérieur de la chambre de four (103),
**caractérisé en ce que**
le four à rouleaux (101) présente un système de rinçage (110) pour l'unité de paliers à roulements (111), qui est réalisé de manière à conduire un gaz de rinçage à travers l'unité de paliers à roulements (111), le gaz de rinçage pouvant être conduit depuis un côté de l'unité de paliers à roulements (111) opposé à la paroi de four (108, 108') à travers l'unité de paliers à roulements (111) et à travers l'ouverture (301) de la paroi de four (108, 108') dans la chambre de four (103) du four à rouleaux (101), et le gaz de rinçage pouvant être fourni à une pression qui est supérieure à une pression à interne dans de la chambre de four (103) du four à rouleaux (101), le système de rinçage (110) comprenant un système de conduite tubulaire (305) pour acheminer le gaz de rinçage à l'unité de paliers à roulements (111) et le système de conduite tubulaire (305) étant disposé au niveau du côté extérieur de la paroi de four (108, 108') du four à rouleaux (101) et l'unité de paliers à roulements (111) comprenant un palier à roulement intérieur (201) à côté duquel est disposé un palier à roulement extérieur (201') de telle sorte que les deux paliers à roulements (201, 201') forment une paire de paliers à roulements, et le palier à roulement intérieur (201) étant disposé plus près du côté extérieur de la paroi de four (108, 108') du four à rouleaux (101) que le palier à roulement extérieur (201'), et **en ce qu'**entre les deux paliers à roulements (201, 201') de la paire de paliers à roulements est réalisée une cavité (302), un élément d'étanchéité (303) étant disposé au niveau d'un côté du palier à roulement extérieur (201') qui est orienté vers la cavité (302) réalisée entre les deux paliers à roulements (201, 201') de la paire de paliers à roulements.

2. Four à rouleaux (101) selon la revendication 1,
**caractérisé en ce**
**qu'**un stabilisateur (109) de la paroi de four (108, 108') peut être utilisé en tant que système de conduite tubulaire (305).

3. Four à rouleaux (101) selon l'une quelconque des revendications précédentes,
**caractérisé en ce que**
le système de conduite tubulaire (305) du système de rinçage (110) est connecté à la cavité (302) de telle sorte que le gaz de rinçage puisse être introduit dans la cavité (302), le gaz de rinçage pouvant ensuite être guidé hors de cette cavité (302) à travers le palier à roulement intérieur (201) et à travers l'ouverture (30) de la paroi de four (108, 108') dans la chambre de four (103) du four à rouleaux (101).

4. Four à rouleaux (101) selon l'une quelconque des revendications précédentes,
**caractérisé en ce que**
le système de rinçage (110) présente un débitmètre (307), le débitmètre (307) étant réalisé de manière à mesurer une vitesse d'écoulement du gaz de rinçage à laquelle le gaz de rinçage peut être conduit à travers le palier à roulement (201, 201') et à travers l'ouverture (301) de la paroi de four (108, 108') dans la chambre de four (103) du four à rouleaux (101).

5. Four à rouleaux (101) selon la revendication 4,
**caractérisé en ce que**
le système de rinçage (110) présente un élément de commande (308), l'élément de commande (308) étant réalisé de manière à commander la vitesse d'écoulement du gaz de rinçage.

6. Four à rouleaux (101) selon l'une quelconque des revendications précédentes,
**caractérisé en ce que**
un gaz de protection est utilisé en tant que gaz de rinçage.

7. Four à rouleaux (101) selon l'une quelconque des revendications précédentes,
**caractérisé en ce que**
de l'air peut être utilisé en tant que gaz de rinçage, cet air pouvant être purifié et/ou séché.

8. Procédé pour chauffer des pièces (102) dans un four à rouleaux (101), le four à rouleaux (101) comprenant au moins une chambre de four (103) pour recevoir les pièces (102), avec au moins deux parois de four latérales (108, 108') et au moins un rouleau de transport (106) étant disposé à l'intérieur de la chambre de four (103) pour le transport des pièces (102) à travers la chambre de four (103), les parois de four (108, 108') présentant à chaque fois au moins une ouverture (301) à travers laquelle le rouleau de transport (106) est guidé et à chaque fois au moins une unité de paliers à roulements (111) étant disposée au niveau d'un côté extérieur des parois de four (108, 108'), laquelle reçoit et supporte le rouleau de transport (106) à l'extérieur de la chambre de four (103),
**caractérisé en ce**
**qu'**un gaz de rinçage est guidé au moyen d'un système de rinçage (110) à travers l'unité de paliers à roulements (111), le gaz de rinçage étant guidé depuis un côté de l'unité de paliers à roulements (111) opposé à la paroi de four (108, 108') à travers l'unité de paliers à roulements (111) et à travers la paroi de four (108, 108') dans la chambre de four (103) du four à rouleaux (101) et le gaz de rinçage étant fourni à une pression qui est supérieure à une pression interne dans la chambre de four (103) du four à rouleaux (101), le système de rinçage (110) comprenant un système de conduite tubulaire (305) pour acheminer le gaz de rinçage à l'unité de paliers à roulements (111) et le système de conduite tubulaire (305) étant disposé au niveau du côté extérieur de la paroi de four (108, 108') du four à rouleaux (101), l'unité de paliers à roulements (111) comprenant un palier à roulement intérieur (201) à côté duquel est disposé un palier à roulement extérieur (201') de telle sorte que les deux paliers à roulements (201, 201') forment une paire de paliers à roulements, et le palier à roulement intérieur (201) étant disposé plus près du côté extérieur de la paroi de four (108, 108') du four à rouleaux (101) que le palier à roulement extérieur (201'), et **en ce qu'**entre les deux paliers à roulements (201, 201') de la paire de paliers à roulements est réalisée une cavité (302), un élément d'étanchéité (303) étant disposé au niveau d'un côté du palier à roulement extérieur (201') qui est orienté vers la cavité (302) réalisée entre les deux paliers à roulements (201, 201') de la paire de paliers à roulements.
